# EUROPEAN PATENT APPLICATION

(11) **EP 3 970 766 A1**
(43) Date of publication of application: **23.03.2022**
(21) Application number: 20805487.4
(22) Date of filing: 07.05.2020
(51) Int. Cl.: A61M 5/142, A61M 5/152, A61B 17/42

(54) **LIQUID MEDICINE SELF-INJECTION SYSTEM**

(30) Priority: 14.05.2019 JP 2019091104
(71) Applicant: Aubex Corporation, Tokyo 130-0026 (JP)
(72) Inventor: KADOWAKI Yu, Tokyo 130-0026 (JP); KAWAMURA Eri, Tokyo 130-0026 (JP)
(74) Representative: Branderhorst, Matthijs Pieter Arie
(86) International application number: PCT/JP2020/018531
(87) International publication number: WO 2020/230689

(57) **Abstract**

[Object] To provide a liquid medicine self-injection system that enables patient himself/herself to easily and safely inject (in particular, bolus-infuse) a prescribed amount of liquid medicine for preventing, treating or improving obstetrical pain, in particular, for a painless childbirth.

[Solution] The liquid medicine self-injection system 1 according to the present invention contains a liquid medicine preservation container 2 for storing a liquid medicine, a liquid medicine feeding-out means 3 for feeding out the liquid medicine from the liquid medicine preservation container 2, and a liquid medicine injection device 4 for allowing the liquid medicine fed out from the liquid medicine preservation container 2 to be self-injected, and is configured to be able to use for preventing, treating or improving obstetrical pain, in particular, for a painless childbirth.

Thus, a prescribed amount of liquid medicine can be easily and safely administered to a patient, that is, pregnant woman, in particular, pregnant woman during childbirth, without giving ill effect on an unborn child or using a large-sized device by self-injection at one time.

## Description

### Technical Field

The present invention relates to a liquid medicine self-injection system.

More specifically, the present invention relates to a liquid medicine self-injection system which enables patient himself/herself to easily and safely inject a prescribed amount of liquid medicine for preventing, treating or improving obstetrical pain.

### Background Art

In a conventional obstetric care, an anesthetic or analgesic agent is often prescribed to a patient for relieving pain for a while.

In this case, a liquid medicine containing an anesthetic or analgesic agent is generally injected continuously into the body of the patient by using a catheter.

However, since there are various symptoms or physical conditions in patients, not a few patients complain about a severe pain with the lapse of time in the initial prescription.

For relieving such a pain, it is necessary to temporarily administer a large amount of anesthetic or analgesic agent.

In this case, when patient himself/herself can temporarily inject a large amount of liquid medicine containing an anesthetic or analgesic agent into the body without calling a doctor or a nurse, the load of the patient himself/herself, the doctor and the like is reduced.

As such an injector for temporarily injecting a liquid medicine, an injector, which is provided on intermediate part between a catheter and a preservation container for preserving a usual liquid medicine to be continuously injected, is known.

For example, in Japanese Patent No. 6222562 (Patent Document 1), a liquid medicine injection system is proposed that, enables to adjust a flow rate of a liquid medicine supplied from a liquid medicine preservation container by a flow rate adjusting means to adjust the time (lockout time) until the next self-injection of a prescribed amount of liquid medicine after a self-injection of a prescribed amount of liquid medicine becomes possible.

This liquid medicine injection system is configured by a specific liquid medicine injection device, a liquid medicine preservation container for storing a liquid medicine, and a flow rate adjusting means for adjusting a flow rate of the liquid medicine,
wherein the flow rate adjusting means is connected with the liquid medicine preservation container through a liquid medicine supply tube, and also, connected with the liquid medicine injection device through a liquid medicine filled tube, and
in the filling of the liquid medicine, by adjusting a flow rate of a liquid medicine supplied from the liquid medicine preservation container, the time until the next self-injection of a prescribed amount of liquid medicine after a self-injection of a prescribed amount of liquid medicine becomes possible is adjusted.

### PRIOR ART DOCUMENTS

### PATENT DOCUMENTS

Patent Document 1: Japanese Patent No. 6222562 (Claims, FIG. 1)

### Disclosure of Invention

### Technical Problem

According to the liquid medicine injection system disclosed in Patent Document 1 above, patient himself/herself can inject a large amount of liquid medicine into the body by one shot while injecting continuously very small amount of liquid medicine.

In case of selecting an anesthetic or analgesic agent as the liquid medicine, this liquid medicine has a danger of giving ill effect, such as that a satisfactory anesthetic or analgesic action is not exhibited when the dose thereof is small, and long term anesthetic or analgesic action is offered by excessive injection into the body when the dose thereof is large, on the body of patient.

Therefore, it is required to further improve in the administration of an appropriate dose of anesthetic or analgesic agent in consideration of influence on a body, in particular influence on an unborn child during pregnancy, by self-injection.

Furthermore, when administering anesthetic or analgesic agent to a patient for a painless childbirth, a device for electromechanically controlling the administration thereof is used.

However, such device is large-sized and expensive in itself, and the operation thereof is complicated, and it is required to foster skilled operator.

In addition, there are problems in that excess flow rate may occur due to the erroneous operation in the device and thereby serious health damage may occur to cause death in the worst case.

In light of the foregoing, an object of the present invention is to provide a liquid medicine self-injection system that enables patient himself/herself to easily and safely inject (in particular, bolus-infuse) a prescribed amount of liquid medicine for preventing, treating or improving obstetrical pain, in particular, for a painless childbirth.

### Solution to Problem

To achieve the above-described object, an aspect of the present invention described in claim 1 is a liquid medicine self-injection system that, contains a liquid medicine preservation container for storing a liquid medicine, a liquid medicine feeding-out means for feeding out the liquid medicine from the liquid medicine preservation container, and a liquid medicine injection device for allowing the liquid medicine fed out from the liquid medicine preservation container to be self-injected,
which is used for preventing, treating or improving obstetrical pain.

According to an aspect of the present invention described in claim 2, in the liquid medicine self-injection system described in claim 1,
the liquid medicine is a liquid medicine having anesthetic or analgesic action, and
the liquid medicine self-injection system is for a painless childbirth.

According to an aspect of the present invention described in claim 3, in the liquid medicine self-injection system described in claim 1 or 2,
the liquid medicine feeding-out means is a non-electric liquid medicine feeding-out means.

According to an aspect of the present invention described in claim 4, in the liquid medicine self-injection system described in any one of claims 1 to 3,
the liquid medicine injection device is configured such that one time injection amount of the liquid medicine is set to 1 to 10 mL.

According to an aspect of the present invention described in claim 5, in the liquid medicine self-injection system described in any one of claims 1 to 4,
a flow rate adjusting means for adjusting a flow rate of the liquid medicine is additionally provided.

According to an aspect of the present invention described in claim 6, in the liquid medicine self-injection system described in claim 5,
the flow rate adjusting means is connected with the liquid medicine preservation container through a liquid medicine supply tube, and also, connected with the liquid medicine injection device through a liquid medicine filled tube, and
when filling of the liquid medicine, a flow rate of the liquid medicine supplied from a liquid medicine preservation container is adjustable by the flow rate adjusting means such that the time until the next self-injection of a prescribed amount of liquid medicine after a self-injection of a prescribed amount of liquid medicine becomes possible is 5 to 60 minutes.

According to an aspect of the present invention described in claim 7 is a method for administering a liquid medicine used for preventing, treating or improving obstetrical pain by a self-injection of a patient,
wherein the liquid medicine self-injection system described in any one of claims 1 to 5 is used.

According to an aspect of the present invention described in claim 8, in the method for administering a liquid medicine described in claim 7,
an amount of the liquid medicine injected by the liquid medicine injection device is set to 1 to 10 mL per one injection.

According to an aspect of the present invention described in claim 9, in the method for administering a liquid medicine described in claim 8,
the liquid medicine self-injection system is the liquid medicine self-injection system described in Claim 5, and
the flow rate of the liquid medicine is adjusted such that the time until the next self-injection after the self-injection becomes possible is 5 to 60 minutes.

According to an aspect of the present invention described in claim 10, in the method for administering a liquid medicine described in any one of claims 7 to 9,
the liquid medicine is always injected to the patient in 0 to 30 mL/hour.

### Advantageous Effects of Invention

The liquid medicine self-injection system according to the present invention contains a liquid medicine preservation container for storing a liquid medicine, in particular, a liquid medicine having anesthetic or analgesic action, a liquid medicine feeding-out means for feeding out the liquid medicine from the liquid medicine preservation container, and a liquid medicine injection device for allowing the liquid medicine fed out from the liquid medicine preservation container to be self-injected, and is configured to be able to use for preventing, treating or improving obstetrical pain, in particular, for a painless childbirth.

Thus, the liquid medicine self-injection system has an advantage in which a prescribed amount of liquid medicine can be easily and safely administered to a patient, that is, pregnant woman, in particular, pregnant woman during childbirth, without giving ill effect on an unborn child and without using a large-sized device by self-injection at one time (in particular, by a bolus).

Further, in the liquid medicine self-injection system, the liquid medicine feeding-out means may be configured by a non-electric liquid medicine feeding-out means.

By such a configuration, the liquid medicine feeding-out means can be easily and safely used since a complicated setting is not required, and a power source is not required. Therefore, the liquid medicine self-injection system can be easily carried, and can be used at the time of a disaster, and becomes inexpensive, simple and safe.

Further, in the liquid medicine self-injection system, one time amount of the liquid medicine injected by the liquid medicine injection device may be set to 1 to 10 mL.

Such a configuration allows the liquid medicine self-injection system to be more suitable for a painless childbirth.

Further, the liquid medicine self-injection system may have a flow rate adjusting means for adjusting a flow rate of the liquid medicine.

Such a configuration allows a prescribed amount of liquid medicine to be easily and safely injected certainly into the body of the patient and allows a lockout time to be controlled.

Furthermore, it is possible to provide a method for administering a liquid medicine used for preventing, treating or improving obstetrical pain through a self-injection of a patient by using the liquid medicine self-injection system.

According to the method for administering a liquid medicine, even if the patient is a pregnant woman, in particular, pregnant woman during childbirth, a prescribed amount of liquid medicine used for preventing, treating or improving obstetrical pain (such as a liquid medicine having anesthetic or analgesic action) can be easily and safely administered without giving ill effect on an unborn child in a mother's body by self-injection of the patient at one time (in particular, by a bolus).

In the method for administering a liquid medicine, one time amount of the injected liquid medicine may be set to 1 to 10 mL.

Such a configuration allows the method for administering a liquid medicine to be more suitable for a painless childbirth.

### BRIEF DESCRIPTION OF DRAWINGS

[Fig. 1] FIG. 1 is an approximately explanatory drawing explaining an example of liquid medicine self-injection system according to the present invention.
[Fig. 2] FIG. 2 is an approximately explanatory drawing explaining another example of liquid medicine self-injection system according to the present invention.

### Description of Embodiments

Hereinafter, the liquid medicine self-injection system according to the present invention will be described in detail below with reference to the accompanying drawings.

Note that the present invention is not limited only to examples disclosed herein, and various modifications can be made thereto without changing the gist of the present invention.

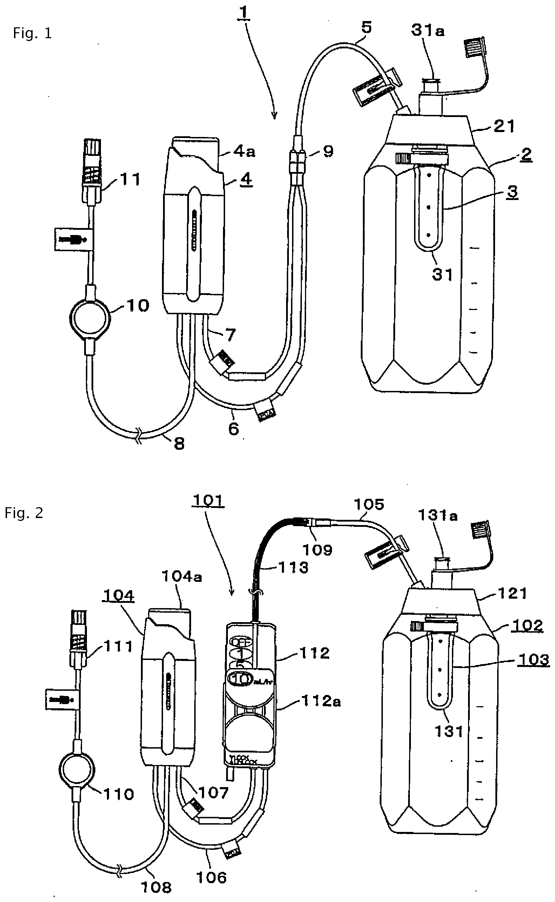

FIG. 1 is an approximately explanatory drawing explaining an example of liquid medicine self-injection system according to the present invention.

In FIG. 1, the liquid medicine self-injection system 1 contains a liquid medicine preservation container 2 for storing a liquid medicine, a liquid medicine feeding-out means 3 for feeding out the liquid medicine from the liquid medicine preservation container 2, and a liquid medicine injection device 4 allowing the liquid medicine fed out from the liquid medicine preservation container 2 to be self-injected by a patient himself/herself.

Note that in the Examples, the liquid medicine self-injection system 1 is configured so as to be usable as a disposable-type system.

A balloon 31 is arranged inside the liquid medicine preservation container 2. The inside of the balloon 31 is filled with a prescribed liquid medicine.

Note that in the Examples, an analgesic agent is selected for the liquid medicine; however, the liquid medicine is not particularly limited as long as the liquid medicine is a liquid medicine used for preventing, treating or improving obstetrical pain. The liquid medicine may be selected from a liquid medicine having anesthetic or analgesic action according to the degree or the like of growth of an unborn child in a mother's body.

As illustrated in FIG. 1, the balloon 31 is arranged inside the liquid medicine preservation container 2 by fixing to a lid member 21 which closes the opening portion of the liquid medicine preservation container 2.

The balloon 31 is comprised of a material having flexibility, such as elastomer, and is configured such that a prescribed liquid medicine is filled inside the balloon from a liquid medicine injection part 31a.

Thus, the balloon 31 is dilated when a liquid medicine is injected inside thereof from the liquid medicine injection part 31a, and the balloon contracts by elastic force of the material. The liquid medicine is fed out by the contraction force to the outside.

That is, the balloon 31 stores and preserves a prescribed liquid medicine, and additionally functions as a liquid medicine feeding-out means for feeding out nonelectrically the liquid medicine to the outside.

Note that the liquid medicine feeding-out means 3 may be a means having any configuration as long as the means has a function for feeding out the liquid medicine from the liquid medicine preservation container 2, in which the liquid medicine is stored and preserved, to the outside. As the liquid medicine feeding-out means, a known liquid medicine feeding-out means, such as pump, or liquid medicine feeding-out means to be developed in future can be used.

As the liquid medicine feeding-out means 3, it is preferable to select a non-electric liquid medicine feeding-out means.

By such a configuration, the liquid medicine feeding-out means 3 can be easily and safely used since a complicated setting is not required, and a power source is not required. Therefore, the liquid medicine self-injection system 1 can be easily carried, and can be used at the time of a disaster, and becomes inexpensive, simple and safe.

The balloon 31 is connected to one end of a liquid medicine tube 5 for supplying a liquid medicine.

On the other hand, the other end of the liquid medicine tube 5 is branched into two flow passages through a connector 9. One flow passage is connected to a liquid medicine filled tube 6, and the other flow passage is connected to a liquid medicine continuous tube 7.

As illustrated in FIG. 1, one end of the liquid medicine continuous tube 7, one end of the liquid medicine filled tube 6, and one end of a liquid medicine injection tube 8 whose other end is connected with a connector 11 through an air vent filter 10 for removing air bubbles are respectively connected to a lower part of the liquid medicine injection device 4. And also, a pressure member 4a, which also serves as a push button, is provided with an upper part of the liquid medicine injection device.

A liquid medicine storage part (not illustrated) for storing a liquid medicine, a pressure member 4a which presses the liquid medicine storage part downward and has a head part also serving as a push button, and a holding means (not illustrated) for holding (pinching) a liquid medicine discharge tube (not illustrated) or the liquid medicine filled tube 6 are arranged inside the liquid medicine injection device 4.

In addition, a transmission member (not illustrated), which acts so as to allow the liquid medicine storage part to be smoothly compressed, is interposed between the liquid medicine storage part and pressure member 4a.

The liquid medicine storage part whose inside can contain a prescribed amount of liquid medicine is configured so as to be deformable by compression and expansion. A liquid medicine filling port and a liquid medicine discharge port are formed on a lower part of the liquid medicine storage part.

The liquid medicine filling port is connected to one end of the liquid medicine filled tube 6, and the liquid medicine discharge port is connected to one end of the liquid medicine discharge tube (not illustrated).

The liquid medicine continuous tube 7 and the liquid medicine injection tube 8 are joined at a joined part (not illustrated) in the inside of the liquid medicine injection device 4, and this joined part is connected to the other end of the liquid medicine discharge tube.

The holding means closes a cross section of the liquid medicine discharge tube in a normal time (in a state where the push button (pressure member 4a) is not pressed) expect the time of additional injection while the holding means closes a cross section of the liquid medicine filled tube 6 at the time of additional injection.

Thus, the liquid medicine flows into the liquid medicine storage part from the liquid medicine filled tube 6 since the cross section of the liquid medicine filled tube 6 is kept opened in a normal time expect the time of additional injection. At the time of additional injection, the cross section of the liquid medicine discharge tube is opened, so that the liquid medicine filling the liquid medicine storage part is discharged through the liquid medicine discharge tube.

Note that the liquid medicine from the liquid medicine continuous tube 7 flows to continuously the liquid medicine injection tube 8 without flowing into the liquid medicine storage part.

At the time, the flow rate of the liquid medicine in the continuous injection may be set to approximately 0 to 30 mL/hour, for example.

As the liquid medicine injection device 4, for example, the liquid medicine injection device disclosed in Japanese Patent No. 6222562 may be used.

That is, the liquid medicine injection device 4 is configured such that the device body thereof contains a liquid medicine storage part whose upper part is opened and whose inside stores a liquid medicine, a pressure member which deforms the liquid medicine storage part by pressurization at the time of additional injection of the liquid medicine to extrude the liquid medicine, a case body which includes a holding member for conducting supply and shut-off of the liquid medicine, and a lid body for closing an opening portion other than the opening; and the liquid medicine storage part has a liquid medicine filled tube, which is for filling the liquid medicine storage part with the liquid medicine, and a liquid medicine discharge tube, which discharges the internal liquid medicine when deformed by pressurization due to the pressure member at the time of the additional injection; and a base end side of the liquid medicine discharge tube has the liquid medicine storage part, and a front end side of the liquid medicine discharge tube has a liquid medicine injection tube for injecting the liquid medicine into a patient and a liquid medicine continuous tube for continuously sending the liquid medicine to the liquid medicine injection tube, through a branch part; and the holding member contains an upper holding member, a tube pressing member and a lower holding member, and also has an elastic body for holding, which energizes the tube pressing member to the upper holding member; and to the liquid medicine storage part the liquid medicine is supplied through the liquid medicine filled tube, and a flow passage of the liquid medicine discharge tube is blocked by the holding member so as not to supply the liquid medicine expect the time of additional injection of the liquid medicine, and the tube pressing member is pushed back against the energizing force of the elastic body by the pressure member to open the flow passage of the liquid medicine discharge tube and discharge the liquid medicine filling the liquid medicine storage part through the liquid medicine discharge tube at the time of the additional injection while the liquid medicine filled tube is held between the tube pressing member, which is pushed back by the pressure member, and the opposing lower holding member to block the flow passage thereof such that the internal liquid medicine does not substantially flow at the time of the additional injection.

Note that the liquid medicine injection device 4 which is for self-injection of a prescribed amount of liquid medicine by a patient may have any configuration as long as the liquid medicine injection device has such function. For the liquid medicine injection device, a known liquid medicine self-injection means or liquid medicine self-injection means to be developed in future may be used.

The liquid medicine injection tube 8 is connected with an injection needle (not illustrated) or the like through the connector 11, and thus the liquid medicine is always injected into the body of the patient.

According to such a liquid medicine injection device 4, in a state where the push button (pressure member 4a) is not pressed, in a so-called normal time, the liquid medicine fed out by the liquid medicine feeding-out means 3 flows to a liquid medicine storage part (not illustrated), which is provided with the liquid medicine injection device 4, through the liquid medicine filled tube 6, and at the same time the liquid medicine continuously flows from liquid medicine continuous tube 7 to the liquid medicine injection tube 8 without flowing into the liquid medicine storage part.

On the other hand, in case that a patient hopes for an additional injection, when pushing the pressure member 4a, the liquid medicine storage part is deformed by pressurization due to the pressure member 4a, so that the liquid medicine filling the liquid medicine storage part is discharged through the liquid medicine discharge tube to inject into the body of the patient through the liquid medicine injection tube 8.

The liquid medicine injection device 4 (in particular, the liquid medicine storage part therein), is configured such that one time injection amount of the liquid medicine is preferably set to 1 to 10 mL, more preferably 2 to 10 mL.

When it is smaller than 1 mL, in case that the liquid medicine to be injected has an anesthetic or analgesic action, the anesthetic or analgesic action tends not to be sufficiently exerted, and when it is larger than 10 mL, in case that the patient is a pregnant woman, there is possibility of giving ill effect on an unborn child.

For the material of each liquid medicine tube used in the present invention, a material which is transparent and flexible, is preferably selected, and elastomer is more preferably selected.

For example, polyolefin (such as LDPE, LLDPE) elastomer, thermoplastic polyurethane elastomer, soft vinyl chloride resin, EVA and the like can be suitably used.

In the present invention, the liquid medicine self-injection system 1 may have a flow rate adjusting means for adjusting a flow rate of the liquid medicine supplied to the liquid medicine injection device 4.

FIG. 2 is an approximately explanatory drawing explaining another example of liquid medicine self-injection system according to the present invention.

In FIG. 2, the liquid medicine self-injection system 101 has the same configuration as the liquid medicine self-injection system 1. In the liquid medicine self-injection system 101, a flow rate adjusting means 112 is connected with a balloon 131 through a liquid medicine supply tube bundle 113, which is configured by a plurality of tubes, and also the flow rate adjusting means is connected to the liquid medicine injection device 104 through the liquid medicine filled tube 106.

In FIG. 2, the other end of the liquid medicine tube 105 is branched into a plurality of flow passages through a connector 109, and one of the liquid medicine supply tube bundle 113 configuring these flow passages is connected to a liquid medicine continuous tube 107, and the remainders are connected to a liquid medicine filled tube 106 through a connector (not illustrated).

The flow rate adjusting means 112 allows the flow rate of the liquid medicine supplied from a liquid medicine preservation container 102 (balloon 131 in FIG. 2) to be adjusted in the filling of the liquid medicine.

For example, the flow rate is set to a prescribed value by a doctor or the like.

In the present invention, for example, the flow rate of the liquid medicine may be set to 10.0 mL/hour or less by the flow rate adjusting means 112.

Additionally, the flow rate adjusting means 112 also allows a time (lockout time) until the next self-injection of a prescribed amount of liquid medicine after a self-injection of a prescribed amount of liquid medicine becomes possible to be adjusted.

For example, the lockout time may be set to approximately 5 to 60 minutes.

Note that the self-injection may be conducted during the lockout time; however, the total maximum amounts of injection are a set amount.

As the flow rate adjusting means 112, an another known flow rate adjusting means or a flow rate adjusting or switching means to be developed in future may be selected as long as a desired flow rate of liquid medicine can be supplied.

Note that in the Examples, the flow rate adjusting means 112, which is configured such that a slider 112a is slid up to a prescribed position to flow a desired flow rate of liquid medicine, has mostly the same configuration as the liquid medicine injection device disclosed in Japanese Utility model registration No. 3185453.

Next, an operation of the liquid medicine self-injection system 101 is explained. Note that an operation of the liquid medicine self-injection system 1 is mostly the same as that of the liquid medicine self-injection system 101.

In the liquid medicine injection device 104 which is in a state where the push button (pressure member 104a) is not pressed, in a so-called normal time, a liquid medicine fed out from the liquid medicine feeding-out means 102 (balloon 131 in FIG. 2) flows to the liquid medicine injection tube 109 from the liquid medicine continuous tube 107 through a joined part without flowing into the liquid medicine storage part provided in the liquid medicine injection device 104.

The liquid medicine injection tube 109 is connected with an injection needle (not illustrated) or the like, and the liquid medicine always flows to a body of a patient.

Furthermore, the liquid medicine, which is supplied from a liquid medicine preservation container 102 due to the liquid medicine feeding-out means 102 (from balloon 131 in FIG. 2), also flows to the liquid medicine storage part through the liquid medicine filled tube 106.

The cross section of the liquid medicine discharge tube, which is connected between the liquid medicine storage part and the joined part, is closed due to the holding means.

Therefore, the liquid medicine is in a difficult situation to flow inside of the liquid medicine storage part.

In case that the patient hopes for an additional injection, when pushing the pressure member 104a, an action of the holding means is blocked, so that the liquid medicine discharge tube is opened to allow the liquid medicine in the liquid medicine storage part to be discharged.

On the other hand, the cross section of the liquid medicine filled tube 106 is closed due to the holding means, so that the liquid medicine is in a difficult situation to flow inside of the liquid medicine filled tube 106.

Simultaneously, when the liquid medicine storage part is compressed by pushing the pressure member 104a, the liquid medicine in the liquid medicine storage part rapidly flows into liquid medicine injection tube 108 through a liquid medicine discharge port and the joined part.

Thus, the liquid medicine, whose amount surpasses a normal inflow amount of the liquid medicine, is injected (bolus-infused) into the body of the patient in a short time.

After the self-injection, the inside of the liquid medicine storage part is filled with the liquid medicine through the liquid medicine filled tube 106 again so as to allow to conduct a next self-injection of a prescribed amount of liquid medicine.

Note that in the Examples, a capacity of the liquid medicine storage part (not illustrated), which is provided with the liquid medicine injection device 104, is set to 3 mL. Therefore, a flow rate of the liquid medicine is set to 6.0 mL/hour by the flow rate adjusting means 112, and additionally a time (lockout time) until the next self-injection of a prescribed amount of liquid medicine becomes possible is set to 30 minutes.

Such a liquid medicine self-injection system enables patient himself/herself to safely and certainly conduct a self-injection of a prescribed amount of liquid medicine when the additional injection of liquid medicine, in particular, a liquid medicine having anesthetic or analgesic action is needed even in case that the patient is a pregnant woman, and there is a concern of an influence on an unborn child.

The liquid medicine self-injection system is suitably used, especially, when the purpose is for a painless childbirth, that is, the patient is a pregnant woman during childbirth.

### Industrial Applicability

The liquid medicine self-injection system according to the present invention enables to easily and certainly administer a liquid medicine, which is additionally injected by a self-injection of a patient, to the patient when needed. The liquid medicine self-injection system gives no influence or an extremely small influence on an unborn child even when the patient is a pregnant woman.

Thus, the present invention may be applied widely in the medicinal industry.

### Reference Signs List

1, 101 Liquid medicine self-injection system
2, 102 Liquid medicine preservation container
21, 121 Lid member
3, 103 Liquid medicine feeding-out means
31, 131 Balloon
31a, 131a Liquid medicine injection port
4, 104 Liquid medicine injection device
4a, 104a Pressure member
5, 105 Liquid medicine tube
6, 106 Liquid medicine filled tube
7, 107 Liquid medicine continuous tube
8, 108 Liquid medicine injection tube
9, 109 Connector
10, 110 Air vent filter
11, 111 Connector
112 Flow rate adjusting means
112a Slider
113 Liquid medicine supply tube bundle

## Claims

1. A liquid medicine self-injection system comprising a liquid medicine preservation container for storing a liquid medicine, a liquid medicine feeding-out means for feeding out the liquid medicine from the liquid medicine preservation container, and a liquid medicine injection device for allowing the liquid medicine fed out from the liquid medicine preservation container to be self-injected,
which is used for preventing, treating or improving obstetrical pain.

2. The liquid medicine self-injection system according to Claim 1, wherein the liquid medicine is a liquid medicine having anesthetic or analgesic action, and
the liquid medicine self-injection system is for a painless childbirth.

3. The liquid medicine self-injection system according to Claim 1 or 2, wherein the liquid medicine feeding-out means is a non-electric liquid medicine feeding-out means.

4. The liquid medicine self-injection system according to any one of Claims 1 to 3, wherein the liquid medicine injection device is configured such that one time injection amount of the liquid medicine is set to 1 to 10 mL.

5. The liquid medicine self-injection system according to any one of Claims 1 to 4, wherein a flow rate adjusting means for adjusting a flow rate of the liquid medicine is additionally provided.

6. The liquid medicine self-injection system according to Claim 5, wherein the flow rate adjusting means is connected with the liquid medicine preservation container through a liquid medicine supply tube, and also, connected with the liquid medicine injection device through a liquid medicine filled tube, and
when filling of the liquid medicine, a flow rate of the liquid medicine supplied from a liquid medicine preservation container is adjustable by the flow rate adjusting means such that the time until the next self-injection of a prescribed amount of liquid medicine after a self-injection of a prescribed amount of liquid medicine becomes possible is 5 to 60 minutes.

7. A method for administering a liquid medicine used for preventing, treating or improving obstetrical pain by a self-injection of a patient,
wherein the liquid medicine self-injection system described in any one of claims 1 to 5 is used.

8. The method for administering a liquid medicine according to Claim 7, wherein an amount of the liquid medicine injected by the liquid medicine injection device is set to 1 to 10 mL per one injection.

9. The method for administering a liquid medicine according to Claim 8, wherein the liquid medicine self-injection system is the liquid medicine self-injection system described in Claim 5, and
the flow rate of the liquid medicine is adjusted such that the time until the next self-injection after the self-injection becomes possible is 5 to 60 minutes.

10. l The method for administering a liquid medicine according to any one of Claims 7 to 9, wherein the liquid medicine is always injected to the patient in 0 to 30 mL/hour.

## Amended claims

### Amended claims under Art. 19.1 PCT

1. (Amended) A liquid medicine self-injection system comprising a liquid medicine preservation container for storing a liquid medicine, a liquid medicine feeding-out means for feeding out the liquid medicine from the liquid medicine preservation container, and a liquid medicine injection device for allowing the liquid medicine fed out from the liquid medicine preservation container to be self-injected,
wherein the liquid medicine feeding-out means is a non-electric liquid medicine feeding-out means, and
which is used for preventing, treating or improving obstetrical pain.

2. The liquid medicine self-injection system according to Claim 1, wherein the liquid medicine is a liquid medicine having anesthetic or analgesic action, and
the liquid medicine self-injection system is for a painless childbirth.

3. (Canceled)

4. (Amended) The liquid medicine self-injection system according to Claim 1 or 2, wherein the liquid medicine injection device is configured such that one time injection amount of the liquid medicine is set to 1 to 10 mL.

5. The liquid medicine self-injection system according to any one of Claims 1 to 4, wherein a flow rate adjusting means for adjusting a flow rate of the liquid medicine is additionally provided.

6. The liquid medicine self-injection system according to Claim 5, wherein the flow rate adjusting means is connected with the liquid medicine preservation container through a liquid medicine supply tube, and also, connected with the liquid medicine injection device through a liquid medicine filled tube, and
when filling of the liquid medicine, a flow rate of the liquid medicine supplied from a liquid medicine preservation container is adjustable by the flow rate adjusting means such that the time until the next self-injection of a prescribed amount of liquid medicine after a self-injection of a prescribed amount of liquid medicine becomes possible is 5 to 60 minutes.

7. A method for administering a liquid medicine used for preventing, treating or improving obstetrical pain by a self-injection of a patient,
wherein the liquid medicine self-injection system described in any one of claims 1 to 5 is used.

8. The method for administering a liquid medicine according to Claim 7, wherein an amount of the liquid medicine injected by the liquid medicine injection device is set to 1 to 10 mL per one injection.

9. The method for administering a liquid medicine according to Claim 8, wherein the liquid medicine self-injection system is the liquid medicine self-injection system described in Claim 5, and
the flow rate of the liquid medicine is adjusted such that the time until the next self-injection after the self-injection becomes possible is 5 to 60 minutes.

10. The method for administering a liquid medicine according to any one of Claims 7 to 9, wherein the liquid medicine is always injected to the patient in 0 to 30 mL/hour.
